Europäisches Patentamt

European Patent Office

Office européen des brevets

(19)

(11) **EP 1 300 695 A1**

(12) **EUROPEAN PATENT APPLICATION**
published in accordance with Art. 158(3) EPC

(43) Date of publication:
**09.04.2003 Bulletin 2003/15**

(21) Application number: **01936965.1**

(22) Date of filing: **12.06.2001**

(51) Int Cl.⁷: **G01T 1/20**, A61B 6/03

(86) International application number:
**PCT/JP01/04950**

(87) International publication number:
**WO 01/096903 (20.12.2001 Gazette 2001/51)**

(84) Designated Contracting States:
**AT BE CH CY DE DK ES FI FR GB GR IE IT LI LU MC NL PT SE TR**

(30) Priority: **12.06.2000 JP 2000175193**

(71) Applicant: **Hitachi Medical Corporation Tokyo 101-0047 (JP)**

(72) Inventor: **YOSHIOKA, Tomonori Ota-ku, Tokyo 144-0052 (JP)**

(74) Representative: **Strehl Schübel-Hopf & Partner Maximilianstrasse 54 80538 München (DE)**

(54) **X-RAY SENSOR AND X-RAY CT APPARATUS COMPRISING IT**

(57)    To provide an X-ray detector for performing highly accurate measurement eliminating measurement errors and a multi-slice X-ray CT apparatus for obtaining a reconstructed image with high diagnosability using said X-ray detector, an X-ray detecting element array is comprised of a scintillator and a light detecting element array with a switching circuit, which is formed with a light detecting element array having light-reception area that is divided in the channel direction and in the slicing direction, and a switching element array for routing a plurality of outputs of said light-reception area divided in the slicing direction, and then connecting these outputs to an external circuit. A switching circuit of said switching element array connects outputs of selected lines of the elements in the same slicing direction on the light detecting element array to an external circuit, and connects the outputs of lines of elements in the slicing direction that are not used for measurement to the common ground potential of said light detecting element array. An X-ray detector is constructed with a plurality of X-ray detecting element arrays polygonally arranged on an arcuate line centered at the focal point of the X-ray tube with a uniform angular pitch in the channel and slicing directions. A plurality of tomograms can be obtained at one time from the data of the intensity of X-rays detected with said X-ray detector.

FIG. 1

EP 1 300 695 A1

## Description

FIELD OF THE INVENTION

The present invention relates to an X-ray detector for detecting X-ray transmission data for a plurality of slices at one time and to an X-ray CT apparatus comprising said detector. More particularly, it relates to an X-ray detector for performing highly accurate measurement, while a measurement circuit is not affected by outputs of photo diode cells that are not used for measurement when a switch array for selecting slice thickness is provided near the photo diode array for converting dose of said transmitted X-ray into electric signals, and to an X-ray CT apparatus that can obtain a reconstructed image with high diagnosability, comprising said X-ray detector.

BACKGROUND OF THE INVENTION

In recent years, the multi-slice X-ray CT apparatus has been attracting attention. In the X-ray CT apparatus, plural lines of X-ray detecting element arrays in the slicing direction are arranged (that is, the X-ray detecting element arrays are two-dimensionally arranged), and two-dimensional X-ray data is collected with one irradiation of X-rays to improve throughput of the apparatus. (Hereafter, an X-ray detector used for said multi-slice X-ray CT apparatus is referred to as a multi-slice X-ray detector.) Compared with the conventional detector with a one line array, (Hereafter, such a detector is referred to as a single-slice X-ray detector and an X-ray CT apparatus using said single-slice detector is referred to as a single-slice X-ray CT apparatus), said multi-slice X-ray CT apparatus has the advantage of better use efficiency of X-rays radiated from the X-ray tube. In said multi-slice X-ray detector, a solid-state detector having scintillators by which high spatial resolution and high S/N (signal/noise) ratio can be obtained is used instead of a conventional ionization chamber type detector using xenon gas. The said solid-state detector comprises a plurality of X-ray detecting elements formed from a scintillator for converting radiated X-rays into light and a light detecting element such as a silicon photo diode for detecting the light produced in said scintillator and outputting it as electric signals, this plurality of said X-ray detecting elements being arranged in an arc around an X-ray source both in the channel direction and in the slicing direction perpendicular to channel direction. In this manner, a plurality of X-ray detecting elements is arranged in the channel direction and in the slicing direction in the multi-slice X-ray detector. Thus, the number of the output signals of said multi-slice X-ray detecting elements become much larger than that of the conventional single-slice X-ray detector, since a large number of X-ray detecting elements is arranged in the slicing and channel directions. It is a pressing object to efficiently extract said output signals in a limited mounting space.

As described above, a multiple-element arrangement is necessary in a detector for an X-ray CT apparatus. Therefore, not a plurality of separate channel chips but a light detecting element array in which a plurality of channels is formed on one chip is used for the light detecting element for converting the output light of the scintillator, said light detecting element array being disclosed in Japanese Patent Laid-open No. 2000-316841.

As the elements of said light detecting element array must be densely arranged in a limited mounting space, semiconductor devices such as a silicon photo diode are generally used for the light detecting element. However, although the light detecting element array used for the conventional single-slice X-ray detector is constructed with a one-dimensional array of receivers and output signal transducers separately only in a single channel direction, the light detecting element array used for the multi-slice X-ray detector needs to be constructed as a two-dimensional array in which the receivers are separated in the channel direction and in the slicing direction perpendicular to the channel direction.

Thus, of course, the number of the output signals per channel increases in accordance with increase of the number of divisions of the light detecting element array in the slicing direction. Further, with a change of slice thickness in the multi-slice X-ray CT apparatus, the width not only of X-ray beams to be radiated but also of the X-ray detecting element in the slicing direction need to be changed. That is, when examining a lesion with an X-ray CT apparatus, the optimum slice thickness depends on the region to be examined, the range of examination, and the objective disease. Therefore, in an X-ray CT apparatus, the slice thickness can be usually selected among several sizes in a range of around 1-10mm, considering the purpose of examination. In a conventional single-slice X-ray detector, the slice thickness for the examination is changed by changing the width of the X-ray beam to be radiated. But, in a multi-slice X-ray detector, the slice thickness of an X-ray beam detected by each X-ray detecting element line cannot be changed only by changing the width of an incident X-ray beam. In the multi-slice X-ray detector, it is necessary not only to change the width of X-ray beams to be radiated but also to change the division of the X-ray detecting element lines in the slicing direction to match the desired slices thickness.

Therefore, to perform measurement with several sizes of slice thickness, it is necessary to change the slice thickness by changing the way in which the outputs of divided photo diode cells are combined, since the X-ray detecting elements are divided among a plurality of photo diode cells as disclosed in said Japanese Patent Laid-open No. 2000-316841.

Thus, it is necessary that the light detecting element array used to provide the multi-slice X-ray detecting elements extensively divided in the slicing direction, and switch arrays are combined so as to connect the output of each division to an external circuit with these divisions combined in the desired manner. Usually, said switch array is placed near the light detecting element array, and they are directly connected with a wire bonding method, as in said Japanese Patent Laid-open No. 2000-316841. And, the output signals of the switch array transmitted to the external circuit from a connector that is mounted on the substrate and connected to a wiring substrate using a wire bonding method. The reason for providing a switching circuit for selecting a slice thickness before the external circuit, that is, a preamplifier for amplifying the output of said light detecting element, is to reduce the total number of said preamplifiers.

The output current of the photo diode elements comprising each X-ray detecting element of the X-ray detector constituted as above is converted into voltage by a current/voltage converter called a preamplifier.

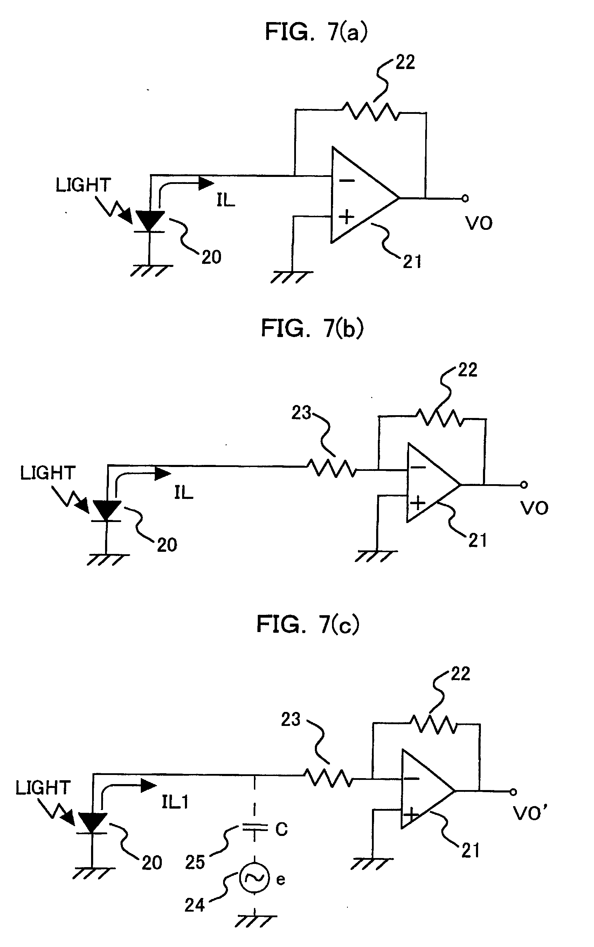

Fig.7(a) is a basic circuit of said current/voltage converter, which is comprised of a photo diode 20 for catching the output light of a scintillator and converting said light into current, an amplifier 21 for amplifying current flowing in said photo diode 20 and a feedback resistor 22 connected between the minus input terminal and output terminal of said amplifier 21, parallel to it. The relation between the input current of said current/voltage converter Ii (A: ampere), the resistance value of the feedback resistor 22 Rf ($\Omega$: ohm), and the output voltage Vo (V: Volt) is indicated with the expression below:

$$Vo = Rf \times Ii \qquad (1)$$

However, there actually exists the internal resistance 23 in the wiring material used to connect the photo diode elements and the amplifier 21. Given that the resistance value of said internal resistance 23 is Ri ($\Omega$: ohm), it works as the input resistance Ri of said amplifier as shown in Fig.7(b).

Even when said internal resistance Ri exists, the relation between the input current Ii and the output voltage Vo is still expressed with the above expression. But, as shown in Fig7(c), there exists a condenser C having some parasitic capacitance between said input resistance Ri and photo diode 20. If voltage e (that is, voltage that is generated when current flows in the photo diode 20 due to the light into which scattered X-rays are converted, or the light leaking from other scintillators, and said current charges the photo diode 20 to junction capacitance) is input through condenser C to amplifier 21 (current/voltage converter), said amplifier works as a voltage amplifier and the voltage Vo' that is Rf/Ri times as large as the applied voltage e is output to the output terminal. As the series internal resistance Ri is usually several 10m$\Omega$ (milliohm) and the feedback resister Rf is several M$\Omega$ (megohm), the amplification factor of voltage of said amplifier 21 becomes as large as $10^6$-$10^8$

times. Then, even faint voltage causes a considerable measurement error. More concretely, the input current of said amplifier is several tens of pA (picoampere)-1µA (microampere) in normal state and the output voltage due to said input current is 5V(volt)-10V. But, if there exists said voltage e, amplifier 21 becomes saturated and its output voltage becomes close to the source voltage of said amplifier 21 (15V, for example), which appears as a considerable measurement error. In the X-ray detector for the multi-slice X-ray CT apparatus, a plurality of photo diode cells are provided in the slicing direction as described above. The output of these cells is routed in a manner appropriate to the slice thickness by changing the setting of the switches, and then this output is transmitted to the measuring circuit. Here, if X-rays enter photo diode cells that are not connected to the measuring circuit, (Even though central rays do not enter them since they are narrowed by the collimator of the radiator in the X-ray tube, some of the central rays become scattered when the central rays are transmitted through an object, and said scattered X-rays enter the photo diode cells that do not perform measurement.) electric charge generated in the photo diode cells that are not connected to the measuring circuit is not discharged outwards, and the additional voltage is given to the electric load. If the voltage thus generated in the photo diode cells that do not perform measurement leaks into a measurement channel through the parasitic capacitance in the wiring, the amplification factor of voltage takes large value as described above, even if the value of said generated voltage is very small. Thus, said generated voltage might cause an error so great with regard to the measured value that it cannot be ignored.

A reconstructed image with high diagnosability cannot be obtained from the measured data having such an error. Here, the objects of the present invention are to provide an X-ray detector for performing highly accurate measurement, not affected by output of photo diode cells that are not used for measurement even when a switch array for selecting slice thickness is provided near the photo diode array of a multi-slice X-ray detector for measuring a plurality of slices at one time, and to provide an X-ray CT apparatus that can obtain an reconstructed image with high diagnosability using the data output by said X-ray detector.

SUMMARY OF THE INVENTION

To achieve the above-mentioned objects, the present invention provides an X-ray detector comprising:

a scintillator for generating light when it detects incident X-rays; and
an X-ray detecting element array with a switching circuit, which comprises an light detecting element array having light-reception area that is divided both in the channel direction and in the slicing direction, and a switching element array for routing the plurality of outputs of said light-reception area that are divided in the slicing direction and transmitting these routed outputs to an external circuit, said scintillator and X-ray detecting element array with a switching circuit being polygonally arranged on an arcuate line centered at the focal point of the X-ray tube with a uniform angular pitch in the channel direction and in the slicing direction,

wherein said switching circuit routes the outputs of selected lines of the elements of the light detecting element array in the slicing direction to an external circuit, and as well leads the output of lines of slicing elements that are not used for measurement at that time to a common ground of said light detecting element array.

When an X-ray detector is constructed in this manner, the photo diode cells used for measurement at the time are not affected by the voltage generated when output terminals of said photo diode cells which are not used for measurement are kept open by connecting output for instance of the photo diode cells used as the light detecting elements, which are not connected at the time to an external circuit, to said ground of the photo diode array. Thus, highly accurate measurement data can be obtained. Also, in said switching circuit, all the rows of channels along the same slicing direction are simultaneously grouped in accordance with a slice thickness selecting signal input from an external circuit and output signals from the cells to said external circuit. Further, said switching circuit comprises a plurality of semiconductor switching elements and a switching element driving signal generating circuit for driving said switching elements. And, connection/disconnection of said switching elements is controlled by said slice thickness selecting signal. By constructing the switching circuit in this manner, the structure of the circuit becomes simple, and easy to connect the output of a cell in a desired position to the external measuring circuit. Further, cells of said light detecting element array and said switching elements are symmetrically arranged. In the cells that are not separated in the combination made to match the slice thickness, output signals of the cells are composed before the cells send said signals to the switching elements. Further, the switching elements symmetrically opposite each other operate in the same way, and are arranged so that the output of each cell is symmetrically transmitted to the external circuit, for the purpose of reducing the total number of said switching elements.

Further, an X-ray CT apparatus of the present invention comprises:

an X-ray tube for radiating an X-ray beam to an object to be examined;
an X-ray detector for detecting X-rays that have transmitted through the object and converting the detected X-rays into electric signals, this X-ray detector being arranged opposite to said X-ray tube;

a preamplifier for amplifying the output signals of said X-ray detector,
an AD converter for converting the analog signals outputted from said preamplifier into digital signals;
a rotating plate which holds at least said X-ray tube and said X-ray detector, this circular plate being driven to rotate around the object;
a scanning condition setting means for setting scanning conditions; and
an image processing means for reconstructing an X-ray image in accordance with the output of said AD converter,

wherein said X-ray detector comprises;

a scintillator for generating light as detecting incident X-rays; and
an X-ray detecting element array with a switching circuit, which comprises an light detecting element array having light-reception area that is divided in the channel direction and in the slicing direction, and a switching element array for routing outputs of said light-reception area that are divided in the slicing direction and leading these outputs to said preamplifier, said scintillator and X-ray detecting element array with a switching circuit being polygonally arranged on an arcuate line around the focal point of the X-ray tube both in the slicing direction and the channel direction with a uniform angular pitch,

wherein said switching circuit leads the output of lines of the slicing elements of the light detecting element array to said preamplifier, said lines being selected according to the slice thickness signal set by the scanning condition setting means, and leads the output of lines of slicing elements that are not used for measurement to common ground of said light detecting element array.

In the thus constructed multi-slice X-ray CT apparatus, photo diodes used as light detecting elements are arranged in an array shape. Further, among the switch arrays that are arranged near said photo diode array for selecting the slice thickness, output terminals of the switch arrays that are not connected to the preamplifier are connected to the ground. Thus, even when electric charge is generated in a photo diode cell due to incidence of scattered X-rays, the disturbance voltage can be prevented from entering the preamplifier that is connected to the switch arrays. Therefore, it is possible to provide a multi-slice X-ray CT apparatus for obtaining a plurality of slice tomograms with high diagnosability, in which errors of measurement data have been eliminated.

Also, all the channels of said switching circuit simultaneously output signals to said preamplifier after combining rows of cells in the same slicing direction according to a slice thickness selecting signal from said scanning condition setting means.

Also, said switching circuit comprises a plurality of semiconductor switching elements and a switching element driving signal generating circuit for driving said switching elements. And, connection/disconnection of said switching elements being controlled by said slice thickness selecting signal. By constructing the switching circuit in this manner, it becomes simple, and easy to connect output of the cells in a desired position to the preamplifier. Further, cells of said light detecting element array and said switching elements are symmetrically arranged. Also, in the cells that are not divided according to the combination corresponding to the selected slice thickness, signals are composed before the cells input these signals to the switching elements. Further, each of said switching elements placed in the above symmetric position operates in the same way, and the output of each cell is symmetrically transferred to said preamplifier for the purpose of reducing the total number of said switching elements.

BRIEF DESCRIPTION OF THE DRAWINGS

Fig. 1 is an external view of an X-ray detecting element array of the present invention.

Fig.2 is a diagram illustrating changes of slice thickness for measurement by combining output of elements that are divided in the slicing direction.

Fig.3 is a diagram of a switching circuit for selecting slice thickness by changing output of a photo diode array, which is a feature of the present invention.

Fig.4 is a diagram illustrating the connection of output of photo diode cells when various slice thicknesses are selected in the present invention.

Fig.5 is a diagram of an X-ray detector in an embodiment of the present invention, mounted on a scanner rotating plate.

Fig.6 is a block diagram illustrating the whole system of a multi-slice X-ray CT apparatus of the present invention.

Fig.7 is a circuit diagram of a current/voltage converting circuit for amplifying signals from the photo diodes.

BEST MODE FOR CARRYING OUT THE INVENTION

Hereafter, an embodiment of a multi-slice X-ray CT apparatus of the present invention will be described with reference to the attached drawings.

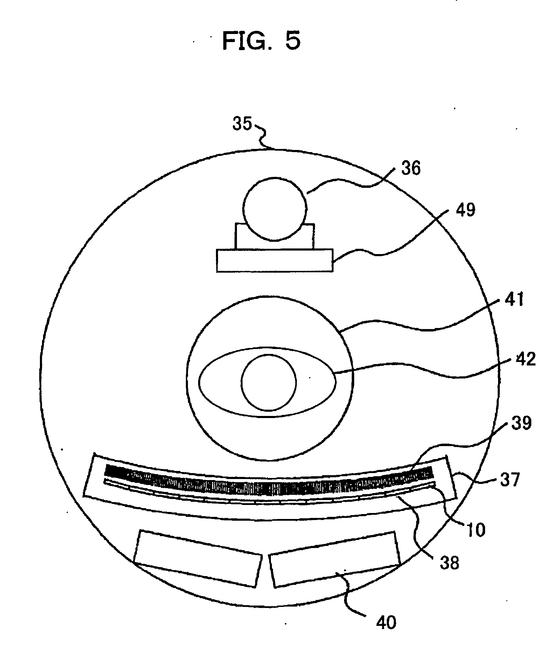

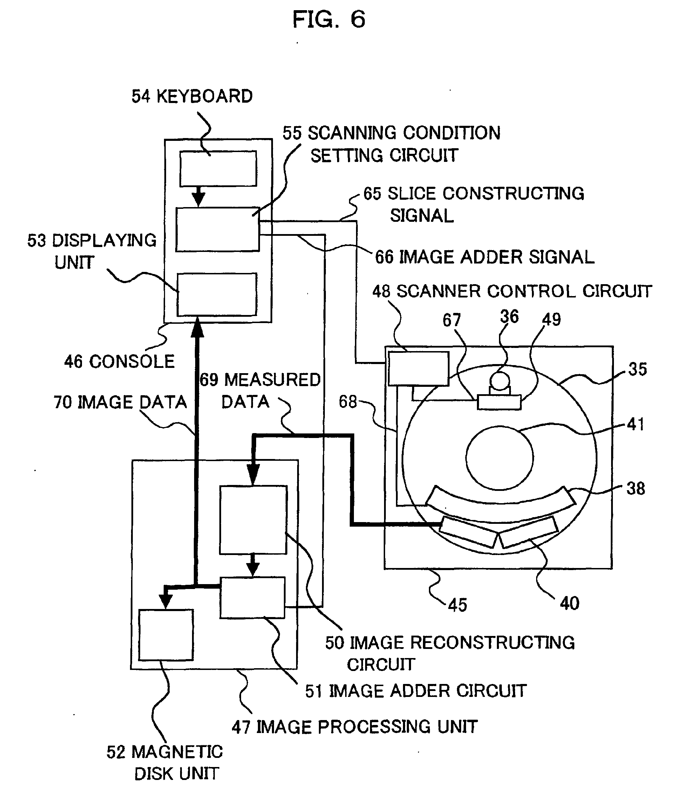

Fig.5 is a diagram of an X-ray detector mounted on a scanner rotating plate in an embodiment of the present invention, and Fig.6 is a block diagram of the whole system of the multi-slice X-ray CT apparatus of the present invention.

In Fig.6, the whole system of a multi-slice X-ray CT apparatus of the present invention comprises three units, that is, a scanner 45, a console 46, and an image processing unit 47. At the center of the scanner 45, a scanner rotating plate 35 is supported so as to freely rotate. On a scanner rotating plate 35, an X-ray tube 36 is mounted opposite to an X-ray detector 38, an opening 41 provided between said X-ray tube 36 and said X-ray detector 38.

With reference to Fig.5, the arrangement of the X-ray detector 38 on the scanner rotating plate 35 will be described. The X-ray detector 38 is arranged opposite to the X-ray tube 36, and between said X-ray detector 38 and said X-ray tube 36 is laid the object 42 inserted in the opening 41 of the scanner rotating plate 35. The X-ray detector 38 and the collimator plates 39 are contained in a detector container 37. The X-ray detector 38 is comprised of a plurality of X-ray detecting element arrays 10, which are polygonally arranged around the focal point of the X-ray tube 36. In front of the X-ray detecting element arrays 10, the collimator plate 39 is radially arranged around the focal point of the X-ray tube 36, said collimator plates 39 provided for preventing scattered X-rays that come from the directions other than the focal point of the X-ray tube is set from entering the X-ray detecting element arrays 10. Also, the X-ray beam collimator 49 is disposed in front of the X-ray tube 36 to narrow an X-ray beam in the slicing direction and in the channel direction. In measurement performed by the X-ray detector 38, the object 42 is inserted into the opening 41, and X-rays are radiated to the object 42 from the focal point of the X-ray tube 36 while the scanner rotating plate 35 is revolved, and the dose of the X-rays transmitted from the each direction to the object 42 is measured. Here, thickness in the slicing direction and width in the channel directions of the X-ray beam radiated from the focal point of the X-ray tube 36 are narrowed by the X-ray beam collimator 49. The output of the X-ray detector 38 is transmitted to an amplifier 40. The output signals are amplified to an adequate level in the amplifier 40, and then they are subject to analog-digital conversion. Then, said converted signals are sent to the image processing unit 47.

In Fig.6, console 46 contains a keyboard 54, a scanning condition setting circuit 55 and an image displaying unit 53, and controls the scanner 45 and displaying a slice image of the object 42.

The scanning conditions of the scanner 45 are input from the keyboard 54 on the console 46 to the scanning condition setting means 55. In this embodiment, a slice construction signal (that is, the information indicating slice thickness $\times$ the number of slices) 65 and an image adding signal 66 are sent to the scanner 45 and the image processing unit 47 respectively, in accordance with information concerning the slice thickness in the scanning conditions.

The slice construction signal 65 is received by a scanner control circuit 48 in the scanner 45. At this time, a collimator opening control signal 67 and a detector switch changing control signal 68 are generated in accordance with said slice construction signal 65, and said signals 67 and 68 are sent to the X-ray beam collimator 49 and the X-ray detector 38 respectively. Then, according to the scanning conditions, the slice thickness of the X-ray beam and the conditions for switching the switching circuit for the signals of the X-ray detecting elements in the slicing direction are set. The outputs of the X-ray detector 38 are amplified and then subject to analog-digital conversion in the amplifier 40, and then it is sent as measured data 69 for a plurality of slices to the image processing unit 47.

The image processing unit 47 includes an image reconstructing circuit 50, an image adder circuit 51, and a magnetic disc unit 52. In a multi-slice X-ray CT apparatus, various tomograms can be obtained by changing slice thickness of the X-ray detector in measurement, and as well by adding reconstructed images. For example, when tomograms of 5mm slice thickness are measured for four pieces, these four tomograms can be dealt with as two tomograms of 10mm slice thickness by combining said four tomograms into two pairs, and utilizing these combined images in diagnosis. In this manner, the image of thick slice width can be used when a lesion is being searched for, and an image of thin slice width can be used when the lesion is being minutely examined. Thus, the efficiency of diagnosis can be improved. Since the number of images obtained by a multi-slice X-ray CT apparatus increases as compared with those obtained with a conventional X-ray CT apparatus, a dedicated image adder circuit 51 is provided in said image processing unit 47. In the image processing device 47, the image reconstructing circuit 50 makes slice images of plural slice locations using the measurement data 69 of corresponding rows of X-ray detecting elements 38 in the slicing direction sent from the scanner 45. Then, said plurality of slice images is sent to image adder circuit 51. The image adder circuit 51 performs adding of the reconstructed slice images, in accordance with the image adder signal 66 sent from the scanning condition setting circuit 55 of the console 46. Final image data 70 thus obtained is sent to the image displaying unit 53 on the console 46, and stored in a memory unit such as magnetic disc unit 52.

Next, the structure of an X-ray detecting element array forming said X-ray detector will be described.

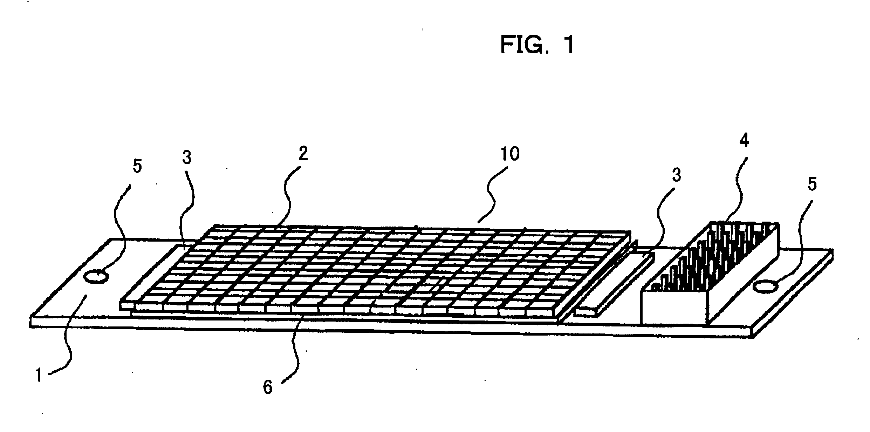

Fig. 1 is an external view of an X-ray detecting element array in an embodiment of the present invention. In Fig.1, the scintillators 2 of the X-ray detecting element array 10 are bonded on the light reception plane of a photo diode array (not shown) which is mounted on a photo diode array substrate, also referred to as a print substrate. The combination of the scintillator 2 and the photo diode 6 is divided with an equal pitch in the channel direction and in the slicing direction to form an array. This combination of scintillator 2 and photo diode 6 comprises an X-ray detecting element. In the figure, 12 elements (for 12 channels) are arranged in the channel direction (longitudinal direction), and 16 elements (for 16 slices) are arranged in the slicing direction (horizontal direction). Thus, the total number of said elements is 192. The output of the photo diode 6 of each X-ray detecting element is sent from the X-ray detecting element array 10 to a connector 4 through a switch array (which is also referred to as a switching circuit). In this manner, said output is connected to an external circuit. And, installation holes 5 are provided on the print substrate 1 for fixing finished X-ray detecting element array 10 on the detector container 37 in Fig.5, by using screws or the like.

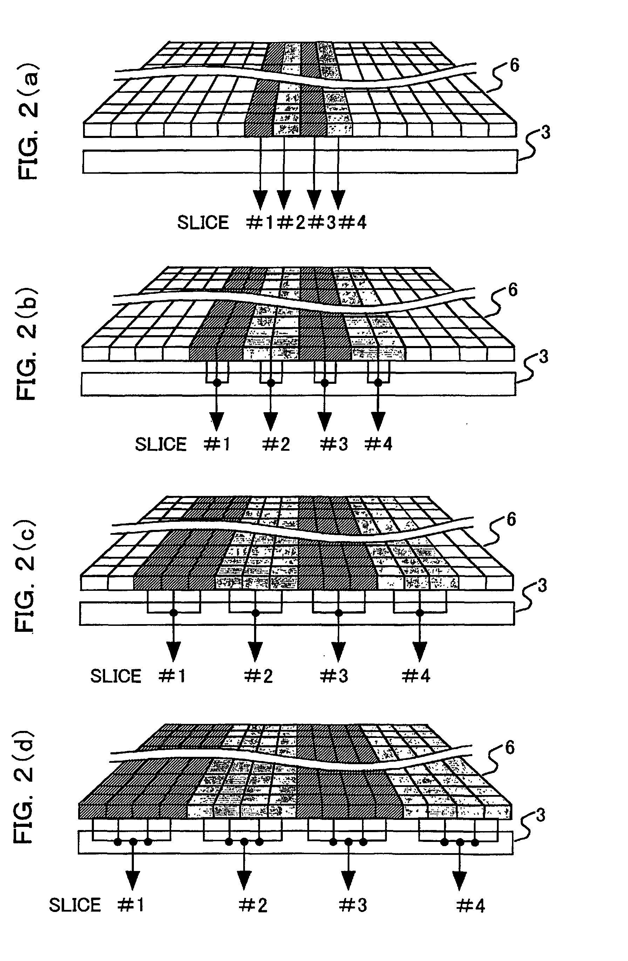

A required number of rows of X-ray detecting elements is arrayed in the X-ray detecting element array 10 in the channel direction, and data for desired slice thickness is measured by changing the combination of outputs of each detecting elements in said switch array 3. Fig. 2 shows an example of changing the slice thickness by switching the combination of the outputs of said divided photo diode cells 6. In the example of Fig.2, four slices of arbitrarily chosen thickness are measured at one time. Fig.2(a) illustrates the case of measuring at one time four slices of "slice thickness 1" corresponding to the basic division pitch of the detecting elements in the slicing direction, separately reading the four slices in the center. Fig.2(b) illustrates the case of measuring at one time four slices of "slice thickness 2" by reading outputs of combined pairs of slices. Similarly, Fig.2(c) and (d) illustrate the case of measuring "slice thickness 3" and "slice thickness 4" respectively.

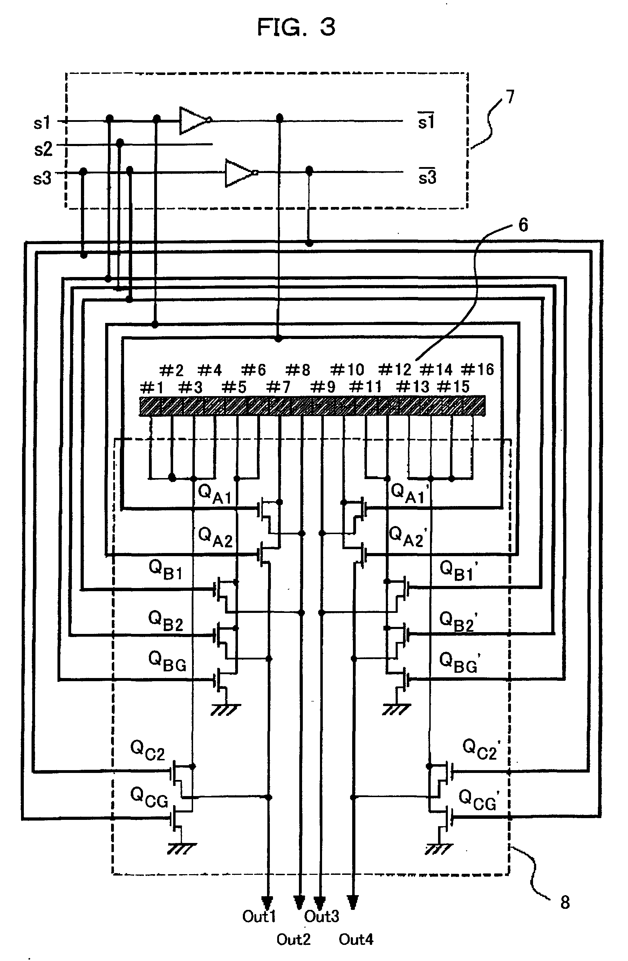

Next, Fig.3 illustrates switch circuit 3, which is the main part of the present invention, for selecting a slice thickness by routing the outputs of the photo diode array. Fig.3 shows the switch circuit for one channel of the photo diode array. A similar switch circuit is provided for each channel. When receiving a slice thickness selection signal (that is, a detector switch changing control signal 68 generated in the scanner control circuit 48 in accordance with the slice constructing signal 65 set by the scanning condition setting circuit in Fig.2) is input from an external circuit, each switching element (which is formed with a field effect transistor is used in this embodiment) operates such that all the channels simultaneously connects the outputs of the combined cells in the same direction to the external circuit. A driving signal for connecting/disconnecting each switching element is generated by a driving signal generating circuit 7 according to said slice thickness selection signal. And the switching circuit 8 provided to receive the output of each photo diode cell is controlled to read outputs of cells in desired positions. In Fig.3, sixteen photo diode cells (#1-#16) are arranged. In accordance with the slice thickness selecting signal, the output of one cell×four lines, two cells×four lines, and four cells× four lines can be read. The photo diode cells and the switching elements are arranged symmetrically. The total number of the switching elements is decreased by sending out the outputs of the cells that do not need to be divided in the combination corresponding to the selected slice thickness before inputting the output signals of the cells to the switching elements. The switching elements placed on a symmetric position operate in the same way, and the outputs (Out1-Out4) of the cells are also symmetrically transmitted to the external circuit. The selection of slice thickness is performed with signal lines s1-s3 described in the upper part of Fig.3. s1 is a signal line for measuring of one cell × four lines, s2 for measuring two cells × four lines, and s4 for measuring four cells × four lines. One of these signal lines is selected, or set to High level, and others are not selected, or set to Low level.

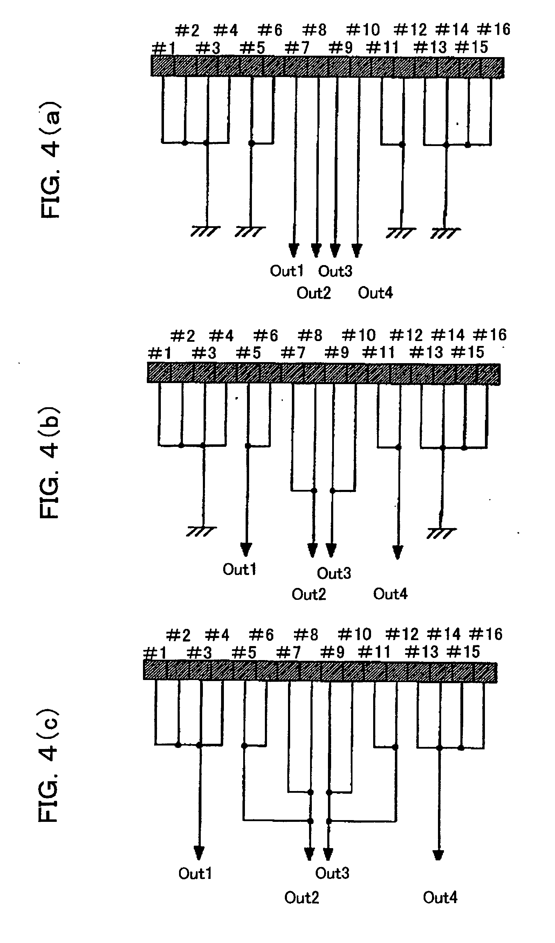

When s1 is selected, that is, s2 and s3 are not selected, the switching elements of $Q_{A2}$, $Q_{A2}'$, $Q_{BG}$, $G_{BG}'$, $Q_{CG}$, $Q_{CG}'$ are connected, and the elements of other cells are not. Thus, the outputs of the cells #7-#10 become Out1-Out4 and are connected to the external circuit. At the same time, the outputs of other cells #1-#6 and #11-#16 are connected to the ground. Consequently, each cell and the signals taken out are operated as described in Fig.4(a).

Next, when s2 is selected, that is, s1 and s3 are not selected, the switching elements of $Q_{A1}$, $Q_{A1}'$, $Q_{B2}$, $Q_{B2}'$, $Q_{CG}$, $Q_{CG}'$ are connected, and other element are not. Thus, the outputs of the cells #5-#12 are combined into pairs and become Out1-Out4 connected to the external circuit. The outputs of other cells #1-#4 and #13-#16 are connected to the ground. Consequently, each cells and the signals to be taken out are operated as described in Fig.4(b).

When s3 is selected, that is, s1 and s2 are not selected, the switching elements of $Q_{A1}$, $Q_{A1}'$, $Q_{B1}$, $Q_{B1}'$, $Q_{C2}$, $Q_{C2}'$ are connected, and other elements are not. Thus, the output of all the cells #1-#16 are combined into four outputs and connected to the external circuit. Consequently, the signals of each cell are connected as described in Fig.4(c). As is clear in Fig.4, all of the cells that are not connected to the measuring circuit due to the above selection are connected to the ground. By constructing the measuring circuit in this manner, even when electric charge is generated when scattered X-rays enter the cells that are not connected to the measuring circuit, voltage is not generated in the output line since said cells that are not connected to the measuring circuit are connected to the ground. Therefore, errors do not occur in the signals going to the measuring circuit.

In this embodiment, the outputs of one cell × four lines, two cells×four lines, and four cells×four lines are read. Also, when the outputs of three cells × four lines or other combinations are read, the outputs of the selected cells can be sent to the measuring circuit by connecting the output of unselected cells to the ground.

By using the thus constructed multi-slice X-ray detector, the dose of the X-rays transmitted from all direction along the circumference of plural slices of object 42 which is put in scanner opening 41 can be measured at one time. In the present invention, output terminals of the switch arrays for selecting slice thickness provided near the photo diode array that are not connected to the external circuit are connected to the ground. Thus, even when electric charge is generated due to scattered X-rays entering the photo diode cells that are not connected to the measuring circuit, disturbance voltage is prevented from entering the amplifier (current/voltage converting circuit) connected to the switch arrays. Thus, errors in measurement data can be avoided. Therefore, by using this X-ray detector in an X-ray CT apparatus, the X-ray CT apparatus for obtaining at one time a plurality of slice tomograms with high diagnosability can be provided.

## Claims

1. An X-ray detector comprising:

   a scintillator which generates light when it detects incident X-rays; and
   an light detecting element array with a switching circuit, which comprises an light detecting element array having light-reception area that are divided in the channel direction and in the slicing direction, and a switching element array for routing a plurality of outputs of said light-reception area that are divided in the slicing direction and connecting these outputs to an external circuit,
   said scintillator and X-ray detecting element array with a switching circuit being polygonally arranged on an arcuate line centered at the focal point of the X-ray tube with a uniform angular pitch in the channel direction and in the slicing direction,

   wherein said switching circuit connects the outputs of selected lines of the elements in the slicing direction of the light detecting element array to an external circuit, and connects the outputs of lines of elements in the slicing direction that are not used for measurement to the common ground potential of said light detecting element array.

2. An X-ray detector according to Claim 1, wherein all the channels of said switching circuit simultaneously output signals to said external circuit, after combining rows of cells in the same slicing direction according to a slicing thickness selecting signal input from an external circuit.

3. An X-ray detector according to Claim 2, wherein said switching circuit comprises a plurality of semiconductor switching elements and a switching element driving signal generating circuit for driving said switching elements. And, connection/disconnection of said switching elements is controlled by said slice thickness selecting signal.

**4.** An X-ray detector according to Claim 3, wherein cells of said light detecting element array and said switching elements are symmetrically arranged, and output signals of the cells that are not divided in the combination corresponding to the selected slice thickness are sent before the cells input said signals to the switching elements, and also, pairs of said switching elements symmetric to each other operate in the same way, and as well the output of all cells are symmetrically connected to the external circuit.

**5.** An X-ray CT apparatus comprising:

an X-ray tube for radiating an X-ray beam to an object to be examined;
an X-ray detector for detecting X-rays that have been transmitted through the object and converting the detected X-rays into electric signals, this X-ray detector being arranged opposite to said X-ray tube;
a preamplifier for amplifying the output signals of said X-ray detector,
an AD converter for converting the analog signals outputted from said preamplifier into digital signals;
a rotating plate for holding at least said X-ray tube and said X-ray detector, this circular plate being driven to rotate around the object;
a scanning condition setting means for setting scanning conditions; and
an image processing means for reconstructing an X-ray image in accordance with the output of said AD converter,

wherein said X-ray detector comprises;

a scintillator which generates light when it detects incident X-rays; and
a light detecting element array with a switching circuit, which comprises an light detecting element array having light-reception area that is divided in the channel direction and in the slicing direction, and a switching element array for routing a plurality of outputs of said light-reception area that are divided in the slicing direction and connecting these outputs to said preamplifier, said scintillator and X-ray detecting element array with a switching circuit being polygonally arranged on an arcuate line centered at the focal point of the X-ray tube with a uniform angular pitch,

wherein said switching circuit connects to said preamplifier the outputs of lines of the elements in the slicing direction of the light detecting element array according to the slice thickness selection signal set by said scanning condition setting means, and connects the output of lines of slicing elements that are not used for measurement to the common ground potential of said light detecting element array.

**6.** An X-ray CT apparatus according to Claim 5, wherein all the channels of said switching circuit simultaneously output signals to said preamplifier after combining rows of cells in the slicing direction according to the slicing thickness selecting signal from said scanning condition setting means.

**7.** An X-ray CT apparatus according to Claim 6, wherein said switching circuit comprises a plurality of semiconductor switching elements and a switching element driving signal generating circuit for driving said switching elements, connection/disconnection of said switching elements being controlled by said slice thickness selecting signal.

**8.** An X-ray CT apparatus according to Claim 7, wherein cells of said light detecting elements and said switching elements are symmetrically arranged, and output signals of cells that are not divided in the combination corresponding to the selected slice thickness are sent before the cells output said signals to the switching elements, and each pair of said switching elements symmetric to each other operates in the same way, and further, the output of each cell is symmetrically connected to said preamplifier.

FIG. 1

EP 1 300 695 A1

FIG. 2(a)

SLICE #1#2#3#4

FIG. 2(b)

SLICE #1 #2 #3 #4

FIG. 2(c)

SLICE #1 #2 #3 #4

FIG. 2(d)

SLICE #1 #2 #3 #4

EP 1 300 695 A1

# FIG. 3

Out1  Out3
Out2  Out4

11

FIG. 4 (a)

#1 #2 #3 #4 #5 #6 #7 #8 #9 #10 #11 #12 #13 #14 #15 #16

Out1  Out3
Out2  Out4

FIG. 4 (b)

#1 #2 #3 #4 #5 #6 #7 #8 #9 #10 #11 #12 #13 #14 #15 #16

Out1  Out3
Out2  Out4

FIG. 4 (c)

#1 #2 #3 #4 #5 #6 #7 #8 #9 #10 #11 #12 #13 #14 #15 #16

Out1  Out3
Out2  Out4

## FIG. 5

# FIG. 6

54 KEYBOARD

55 SCANNING CONDITION SETTING CIRCUIT

65 SLICE CONSTRUCTING SIGNAL

53 DISPLAYING UNIT

66 IMAGE ADDER SIGNAL

48 SCANNER CONTROL CIRCUIT

67  36  49

46 CONSOLE

35

69 MEASURED DATA

70 IMAGE DATA

68

41

38

45  40

50 IMAGE RECONSTRUCTING CIRCUIT

51 IMAGE ADDER CIRCUIT

47 IMAGE PROCESSING UNIT

52 MAGNETIC DISK UNIT

FIG. 7(a)

FIG. 7(b)

FIG. 7(c)

## INTERNATIONAL SEARCH REPORT

| | International application No. |
|---|---|
| | PCT/JP01/04950 |

**A. CLASSIFICATION OF SUBJECT MATTER**
Int.Cl$^7$  G01T1/20, A61B6/03

According to International Patent Classification (IPC) or to both national classification and IPC

**B. FIELDS SEARCHED**

Minimum documentation searched (classification system followed by classification symbols)
Int.Cl$^7$  G01T1/20, A61B6/03

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 11-347023 A (Sony Corporation),<br>21 December, 1999 (21.12.99),<br>Full text; all drawings<br>Full text; all drawings<br>(Family: none) | 1,5<br>2-4,6-8 |
| Y | JP 63-58772 U (Fuji Electric Co., Ltd.),<br>19 April, 1988 (19.04.88),<br>Full text; all drawings<br>(Family: none) | 1,5 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 September, 2001 (04.09.01) | 18 September, 2001 (18.09.01) |

| Name and mailing address of the ISA/ | Authorized officer |
|---|---|
| Japanese Patent Office | |
| Facsimile No. | Telephone No. |

Form PCT/ISA/210 (second sheet) (July 1992)